## Europäisches Patentamt
## European Patent Office
## Office européen des brevets

(11) Veröffentlichungsnummer: **0 104 591**
**B1**

(12) **EUROPÄISCHE PATENTSCHRIFT**

(45) Veröffentlichungstag der Patentschrift:
**18.05.88**

(21) Anmeldenummer: **83109335.6**

(22) Anmeldetag: **20.09.83**

(51) Int. Cl.⁴: **A 61 G 13/00**

(54) **Befestigungsanordnung für Zubehöre für eine Patientenliege.**

(30) Priorität: **29.09.82 DE 3236135**

(43) Veröffentlichungstag der Anmeldung:
**04.04.84 Patentblatt 84/14**

(45) Bekanntmachung des Hinweises auf die Patenterteilung:
**18.05.88 Patentblatt 88/20**

(84) Benannte Vertragsstaaten:
**DE FR SE**

(56) Entgegenhaltungen:
**DE-A-2 759 079**
**FR-A-2 184 165**
**US-A-2 550 306**
**US-A-4 346 298**

(73) Patentinhaber: **Siemens- Elema AB, Röntgenvägen 2, S-171 95 Solna 1 (SE)**
(84) Benannte Vertragsstaaten: **SE**

(73) Patentinhaber: **Siemens Aktiengesellschaft Berlin und München, Wittelsbacherplatz 2, D-8000 München 2 (DE)**
(84) Benannte Vertragsstaaten: **DE FR**

(72) Erfinder: **Westerberg, Hans, Lummergangen 16, S-13 535 Tyresoe (SE)**
Erfinder: **Leandersson, Enar, Oxbergsvaegen 25, S-17 800 Ekeroe (SE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Die Erfindung betrifft eine Befestigungsanordnung gemäss dem ersten Teil des Anspruches 1.

Eine Befestigungsanordnung, die an das Kopfende der Patientenliege appliziert ist, ist durch den SIEMENS-Prospekt "KOORDINAT 3D" bekannt. Diese Befestigungsanordnung wird verwendet, wenn im Brust- und Bauchbereich multidirektionale Aufnahmen, d.h. Schrägaufnahmen aus mehreren Richtungen, gemacht werden sollen. Bei einer solchen Röntgenuntersuchung darf in dem Bereich kein Zubehör vorhanden sein, da es die Aufnahmen verschlechtern und die Handhabung der Geräte stören würde. Die bekannte Befestigungsanordnung besteht aus einer Holzplatte und weist Schienen auf, die auf die röntgenstrahlendurchlässige Platte aufgeschoben werden können. Diese Befestigungsanordnung besitzt auch Löcher zum Applizieren von Zubehör wie z. B. Armstützen, Handgriffen, Steuervorrichtungen, Druckrezeptoren und Behältern für Kochsalzlösung.

Der Nachteil dieser Befestigungsanordnung besteht darin, dass sie keine Standardschienen zum Befestigen von Zubehör besitzt. Das ansonsten auf den Standardschienen applizierte Zubehör muss daher teilweise neu konstruiert werden, damit es in den Löchern befestigt werden kann. Weiterhin verläuft die bekannte Platte unter dem röntgenstrahlentransparenten Kopfteil der Patientenliege, so dass dieser Bereich bei einer applizierten Platte nicht störungsfrei aufgenommen werden kann.

Eine ähnliche Befestigungsanordnung der eingangs genannten Art, die an dem Kopfende der Patientenliege anbringbar ist, ist durch die US-A-4 346 298 bekannt. Der Bügel weist Nuten auf, in die Zapfen einschiebbar sind, die lediglich als Halterungen für ein Kopfkissen dienen. Auch diese Befestigungsanordnung weist den Nachteil auf, dass sie keine Standardschienen zum Befestigen von Zubehör besitzt.

Der Erfindung liegt die Aufgabe zugrunde, eine Befestigungsanordnung der eingangs genannten Art zu schaffen, an der Zubehör mit Standard-Befestigungen appliziert werden und ausserdem auch bei an der Platte arretierter Befestigungsanordnungen Röntgenaufnahmen in diesem Bereich gemacht werden können.

Diese Aufgabe ist erfindungsgemäss durch die Merkmale des zweiten Teiles des Anspruches 1 gelöst. Dadurch, dass der Bügel im Randbereich der Patientenliege verläuft, kann auch der vom Bügel umspannte Bereich störungsfrei aufgenommen werden. Ferner kann durch die Schiene bzw. die Schienen Zubehör mit Standardbefestigungen appliziert werden.

Weitere Vorteile und Einzelheiten der Erfindung ergeben sich aus den Unteransprüchen.

Die Erfindung ist nachfolgend anhand eines in der Zeichnung dargestellten Ausführungsbeispieles näher erläutert. Es zeigen:

FIG 1 eine Befestigungsanordnung nach der Erfindung

FIG 2 einen Schnitt durch eine Befestigungsanordnung gemäss der Schnittlinie II-II von Fig. 1 und

Fig. 3 eine Perspektivansicht eines Röntgenuntersuchungsgerätes mit einer Befestigungsanordnung nach Fig. 1.

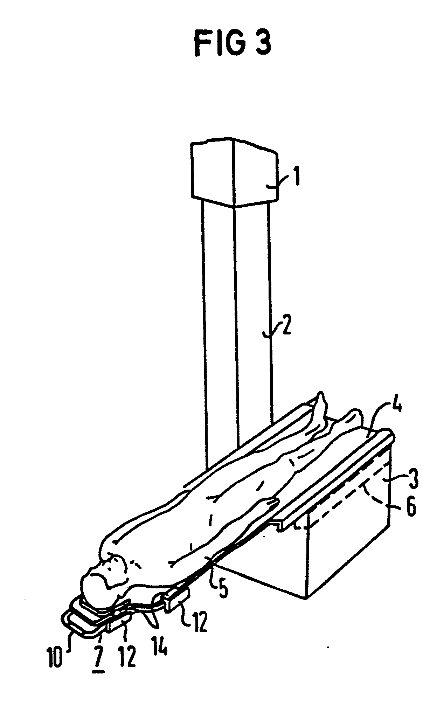

In Fig. 3 ist ein Röntgenuntersuchungstisch gezeigt, der eine Teleskopsäule 1 besitzt, an derem ausfahrbaren Arm 2 ein Untergestell 3 mit einer Patientenliege aus einer rahmenlosen, röntgenstrahlendurchlässigen Platte 4 für einen Patienten 5 gelagert ist. Das Untergestell 3 weist in Tischlängsrichtung verschiebbare Balken 6 auf, die hier nicht dargestellte Schienen für die Befestigung von Zubehören tragen. In dieser Figur sind die Balken 6 in ihren Parklagen dargestellt. Wenn im Bereich des Kopfes oder der Schultern des Patienten Röntgenaufnahmen, insbesondere Schrägaufnahmen gemacht werden sollen, werden die Balken 6 mit ihren Schienen aus dem Untergestell 3 herausgezogen, so dass die notwendigen Zubehöre darauf befestigt werden können. Die Aufnahmen werden hierdurch nicht beeinflusst.

Wenn multidirektionale Aufnahmen im Brust- oder Bauchbereich des Patienten vorgenommen werden sollen, werden die Balken 6 in das Untergestell 3 hineingeschoben. Die bekannten und daher nicht dargestellten Zubehöre werden nun auf Standardschienen 12 einer später näher beschriebenen Befestigungsanordnung 7, die am Kopfende angebracht wird, befestigt. Der Brust- und Bauchbereich ist nun frei von Balken und Zubehören, so dass einwandfreie Röntgenaufnahmen in beliebigen Winkeln gemacht werden können.

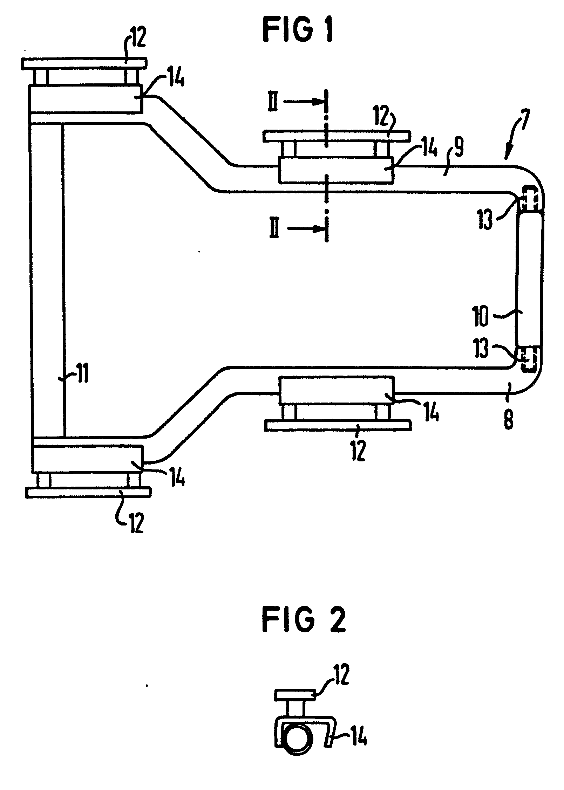

In Fig.1 ist gezeigt, dass die Befestigungsanordnung 7 aus einem der Kontur des Kopfbereiches der Platte 4 angepassten Bügel besteht. Der Bügel weist zwei Seitenteile 8, 9 auf, die an einem Ende starr und am anderen über eine Spannvorrichtung, z. B. eine Wantschraube 10 miteinander verbunden sind. Die starre Verbindung, die eine Fortsetzung des Bügels bildet, besteht aus einer Leiste 11 aus einem röntgendurchlässigen Material. Die Leiste 11 verläuft in applizierter Lage der Befestigungsanordnung 7 unterhalb der Platte 4 und weist eine Form auf, die dem Plattenprofil entspricht. An den Seitenteilen 8, 9 des Bügels sind Klammern 14 zum Umgreifen der Platte 4 befestigt (Fig. 2). Ferner sind an den Seitenteilen Schienen 12 für die Befestigung von Zubehören angebracht.

Der Abstand zwischen den Seitenteilen 8, 9 ist so bemessen, dass der Bügel mühelos auf den Kopfteil der Platte 4 aufgeschoben werden kann. Beim Drehen der Wantschraube 10, die über Gewindebolzen 13 mit den Seitenteilen 8, 9 in Eingriff steht, werden diese an die Aussenkontur der Platte 4 gedrückt, wodurch der Bügel an dieser befestigt wird. Beim Lösen des Bügels von der Platte 4 wird die Wantschraube 10 in die entgegengesetzte Richtung gedreht, wobei die Seitenteile 8, 9 auseinander gehen.

Die Form der Befestigungsanordnung lässt den vom Bügel umfassten Bereich der röntgenstrahlendurchlässigen Platte 4 frei von eine Röntgenaufnahme störenden Materialien. Dadurch können im Bereich der Schultern bzw. des Kopfes des Patienten auch bei einer applizierten Befestigungsanordnung 7 Röntgenaufnahmen einwandfrei vorgenommen werden.

## Patentansprüche

1. Befestigungsanordnung für Zubehör, die an einer rahmenlosen röntgenstrahlendurchlässigen Platte (4) einer Patientenliege arretierbar ist, wobei die Befestigungsanordnung aus einem zwei Seitenteile (8, 9) aufweisenden und der Aussenkontur eines Bereiches der Platte angepassten Bügel (8, 9, 11) besteht, der die Platte (4) im Randbereich zumindest teilweise umgreift, dadurch gekennzeichnet, dass die Seitenteile (8, 9) an einem Ende starr und am anderen über eine Spannvorrichtung (10) zum Arretieren des Bügels (8, 9, 11) an der Platte (4) miteinander verbunden sind und dass der Bügel (8, 9, 11) mindestens eine Schiene (12) zum Befestigen des Zubehörs aufweist.

2. Befestigungsanordnung nach Anspruch 1, dadurch gekennzeichnet, dass die starre Verbindung des Bügels (8, 9, 11) aus einer derart ausgebildeten Leiste (11) aus röntgenstrahlendurchlässigem Material besteht, dass sie unterhalb der Platte (4) verläuft, wenn der Bügel (8, 9, 11) an dieser angebracht ist.

3. Befestigungsanordnung nach Anspruch 1 oder 2, dadurch gekennzeichnet, dass die starre Verbindung (11) eine Fortsetzung des Bügels (8, 9, 11) bildet.

4. Befestigungsanordnung nach einem der Ansprüche 1 bis 3, dadurch gekennzeichnet, dass als Spannvorrichtung (10) eine Spannschraube vorgesehen ist.

5. Befestigungsanordnung nach einem der Ansprüche 1 bis 4, dadurch gekennzeichnet, dass die Seitenteile des Bügels (8, 9, 11) Klammern (14) zum Umgreifen der Platte (4) aufweisen.

## Claims

1. A securing arrangement for an attachment, which securing arrangement can be fixed at a frameless X-ray-permeable plate (4) of a patient couch, the securing arrangement consisting of a shackle (8, 9, 11) which exhibits two side parts (8, 9) and which is adapted to the external contour of a region of the plate (4), and which at least partially grips round the plate (4) in the edge region, characterised in that the side parts (8, 9) are connected to one another at one end rigidly and at the other by means of a clamping device (10) for fixing the shackle (8, 9, 11) to the plate (4), and in that the shackle (8, 9, 11) exhibits at least one rail (12) to secure the attachment.

2. A securing arrangement according to Claim 1, characterised in that the rigid connection of the shackle (8, 9, 11) consists of a strip (11) constructed of X-ray-permeable material, and in such a manner that it extends beneath the plate (4) when the shackle (8, 9, 11) is fitted to the latter.

3. A securing arrangement according to Claim 1 or 2, characterised in that the rigid connection (11) forms a continuation of the shackle (8, 9, 11).

4. A securing arrangement according to one of Claims 1 to 3, characterised in that a clamping screw is provided as clamping device (10).

5. A securing arrangement according to one of Claims 1 to 4, characterised in that the side parts of the shackle (8, 9, 11) exhibit clamps (14) for gripping round the plate (4).

## Revendications

1. Dispositif de fixation pour des accessoires qui sont susceptibles d'être fixés au plateau d'un support de patient, qui est dépourvu de cadre et qui est transparent aux rayons X, ledit dispositif de fixation étant constitué par un étrier (8, 9, 11) comprenant deux éléments latéraux (8, 9) et étant adapté au contour extérieur d'une partie du plateau (4), caractérisé par le fait que les éléments latéraux (8, 9) sont reliés rigidement entre eux à une extrémité, alors qu'ils sont, à l'autre extrémité, reliés entre eux par l'intermédiaire d'un dispositif de serrage (10) pour la fixation de l'étrier (8, 9, 11) au plateau (4), et que l'étrier (8, 9, 11) comporte au moins un rail (12) pour la fixation des accessoires.

2. Dispositif de fixation selon la revendication 1, cararctérisé par le fait que la liaison rigide de l'étrier (8, 9, 11) est constituée par une réglette (11) réalisée de telle façon avec un matériau transparent aux rayons X qu'elle s'étend sous le plateau (4) lorsque l'étrier (8, 9, 11) y est fixé.

3. Dispositif de fixation selon la revendication 1 ou 2, caractérisé par le fait que la liaison rigide (11) constitue le prolongement de l'étrier (8, 9, 11).

4. Dispositif de fixation selon l'une des revendications 1 à 3, caractérisé par le fait que l'on prévoit, comme dispositif de serrage (10), une vis de serrage.

5. Dispositif de fixation selon l'une des revendications 1 à 4, caractérisé par le fait que les éléments latéraux de l'étrier (8, 9, 11) comportent des crochets (14) pour enserrer le plateau (4).

FIG 1

FIG 2

# FIG 3